# EUROPÄISCHE PATENTSCHRIFT

(11) **EP 0 707 010 B1**
(45) Veröffentlichungstag und Bekanntmachung des Hinweises auf die Patenterteilung: **05.12.2001**
(21) Anmeldenummer: 95115279.2
(22) Anmeldetag: 28.09.1995
(51) Int. Cl.: C07F 17/00, C08F 10/00

(54) **Metallocenverbindung**
Metallocenes
Métallocènes

(30) Priorität: 10.10.1994 DE 4436113
(43) Veröffentlichungstag der Anmeldung: 17.04.1996
(73) Patentinhaber: Basell Polyolefine GmbH, 67056 Ludwigshafen (DE)
(72) Erfinder: Herrmann, Hans-Friedrich, Dr., D-64521 Dornheim (DE); Aulbach, Michael, Dr., D-65719 Hofheim (DE); Küber, Frank, Dr., D-61440 Oberursel (DE)

(56) Entgegenhaltungen:
- EP-A- 0 549 900
- CHEMICAL ABSTRACTS, vol. 117, no. 15, 12. Oktober 1992 (1992-10-12) Columbus, Ohio, US; abstract no. 150662, ALBRECHT, KARSTEN ET AL: "Domino-Heck coupling of aryl halides and dicyclopentadiene: a simple access to polycyclic arene-annulated cyclopentadienes as ligands for meta complexes" XP002110837 & SYNLETT (1992), (6), 521-3 ,1992,

## Beschreibung

Die vorliegende Erfindung bezieht sich auf eine Metallocenverbindung, welche sich als Katalysatorkomponente zur Olefinpolymerisation eignet. Die Erfindung bezieht sich außerdem auf ein Verfahren zur Herstellung von Polyolefinen unter Verwendung der erfindungsgemäßen Metallocenverbindung.

Es sind verbrückte Metallocene bekannt (EP 284 708, EP 344 887). Bei deren Synthese entsteht neben der gewünschten rac-Verbindung gleichermaßen auch die meso-Verbindung, welche bei der Polymerisation von 1-Olefinen nicht zur Herstellung isotaktischer Polymere geeignet ist und welche generell eine deutlich geringere Polymerisationsaktivität zeigt. Zur Herstellung hochwertiger Polyolefin-Werkstoffe ist daher die aufwendige Abtrennung der meso-Form erforderlich.

Weiterhin sind Verfahren bekannt zur Herstellung von Polyethylen und Ethylen-1-Olefin-Copolymeren unter Verwendung von stereorigiden Metallocenen mit verbrückten, substituierten Cyclopentadienylliganden (EP 399348). Dieses Konzept wurde in WO 9324536 bei Verwendung zweifach verbrückter Metallocene zur Herstellung von (Co-)Polymeren fortentwickelt.

Weiterhin sind unverbückte Metallocene bekannt zur Herstellung von Polyolefinen (EP 69951, EP 129368, EP 170059, EP 206794, EP 285443 oder EP 294942). Diese unverbrückten Metallocene sind insbesondere Cyclopentadienylzirkonkomplexe, deren Cyclopentadienylreste substitutiert oder unsubstituiert sind. Nachteilig bei diesen unverbrückten Metallocenen ist die niedrige Comonomereinbaurate sowie daß bei der Herstellung von Copolymeren niedriger Dichte hohe Comonomer-Konzentrationen erforderlich sind.

Es bestand somit die Aufgabe eine Metallocenverbindung zur Verfügung zu stellen, welche die Nachteile des Standes der Technik vermeidet.

Es wurde nun gefunden, daß diese Aufgabe gelöst wird durch eine spezielle unverbrückte Metallocenverbindung.

Die vorliegende Erfindung bezieht sich somit auf eine Metallocenverbindung der Formel (I) 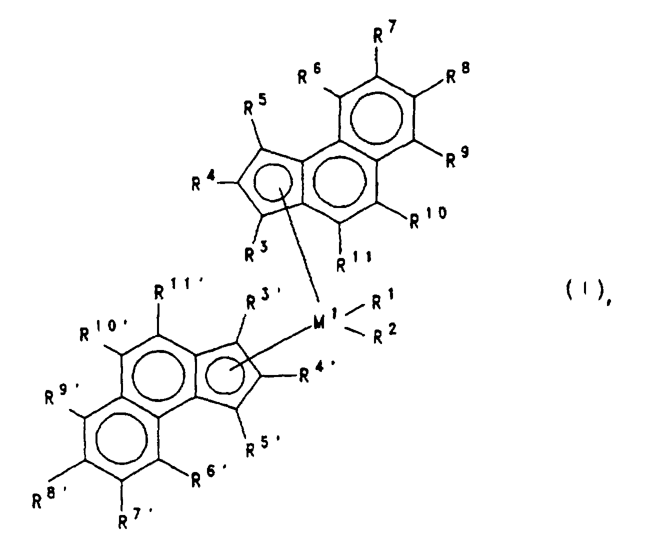 worin
M¹ ein Metall der Gruppe IVb, Vb oder Vlb des Periodensystems ist,
R¹ und R² gleich oder verschieden sind und ein Wasserstoffatom, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₈-C₄₀-Arylalkenylgruppe, eine OH-Gruppe oder ein Halogenatom bedeuten,
die Reste R³, R⁴, R⁵, R⁶, R⁷, R⁸, R⁹, R¹⁰ und R¹¹ gleich oder verschieden sind und ein Wasserstoffatom, ein Halogenatom, einen C₁-C₂₀-Kohlenwasserstoffrest, der halogeniert sein kann, einen -NR₂, -SR, -OR, -OSiR₃, -SiR₃ oder PR₂-Rest bedeuten, worin R ein Halogenatom, eine C₁-C₁₀-Alkylgruppe oder eine C₆-C₁₀-Arylgruppe ist, oder zwei oder mehr der Reste R³, R⁴, R⁵, R⁶, R⁷, R⁸, R⁹, R¹⁰ und R¹¹ zusammen mit den sie verbindenden Atomen ein Ringsystem bilden, und
die Reste R^{3'}, R^{4'}, R^{5'}, R^{6'}, R^{7'}, R^{8'}, R^{9'}, R^{10'} und R^{11'} gleich oder verschieden sind und ein Wasserstoffatom, ein Halogenatom, einen C₁-C₂₀-Kohlenwasserstoffrest, der halogeniert sein kann, einen -NR₂, -SR, -OR, -OSiR₃, -SiR₃ oder PR₂-Rest bedeuten, worin R ein Halogenatom, eine C₁-C₁₀-Alkylgruppe oder eine C₆-C₁₀-Arylgruppe ist, oder zwei oder mehr der Reste R^{3'}, R^{4'}, R^{5'}, R^{6'}, R^{7'}, R^{8'}, R^{9'}, R^{10'} und R^{11'} zusammen mit den sie verbindenden Atomen ein Ringsystem bilden.

Bevorzugt ist M¹ ein Metall der Gruppe IVb wie Titan, Zirkonium oder Hafnium. R¹ und R² sind bevorzugt gleich oder verschieden, bevorzugt gleich, und eine C₁-C₁₀-Alkylgruppe, eine C₇-C₁₅-Arylalkylgruppe oder ein Halogenatom wie Fluor, Chlor, Brom oder Jod, insbesondere Chlor.
Die Reste R³, R⁴, R⁵, R⁶, R⁷, R⁸, R⁹, R¹⁰ und R¹¹ sind gleich oder verschieden und bevorzugt ein Wasserstoffatom, ein C₁-C₂₀-Kohlenwassserstoffrest wie eine C₁-C₂₀-Alkylgruppe, eine C₆-C₁₄-Arylgruppe, eine C₂-C₂₀-Alkenylgruppe, eine C₇-C₄₀-Arylalkylgruppe oder eine C₈-C₄₀-Arylalkenylgruppe, oder zwei oder mehr der Reste R³, R⁴, R⁵, R⁶, R⁷, R⁸, R⁹, R¹⁰ und R¹¹ bilden zusammen mit den sie verbindenden Atomen ein Ringsystem.
Die Reste R^{3'}, R^{4'}, R^{5'}, R^{6'}, R^{7'}, R^{8'}, R^{9'}, R^{10'} und R^{11'} sind bevorzugt gleich oder verschieden und ein Wasserstoffatom, ein C₁-C₂₀-Kohlenwassserstoffrest wie eine C₁-C₂₀-Alkylgruppe, eine C₆-C₁₄-Arylgruppe, eine C₂-C₂₀-Alkenylgruppe, eine C₇-C₄₀-Arylalkylgruppe oder eine C₈-C₄₀-Arylalkenylgruppe, oder zwei oder mehr der Reste R^{3'}, R^{4'}, R^{5'}, R^{6'}, R^{7'}, R^{8'}, R^{9'}, R^{10'} und R^{11'} bilden zusammen mit den sie verbindenden Atomen ein Ringsystem.

Besonders bevorzugt sind Verbindungen der Formel 1, worin M¹ ein Metall der Gruppe IVb, insbesondere Zirkonium ist.

R¹ und R² sind gleich oder verschieden, insbesondere gleich und sind besonders bevorzugt eine C₁-C₁₀-Alkylgruppe wie Methyl, Ethyl, Propyl, Butyl oder Heptyl oder ein Halogenatom, wie Chlor.
R³ und R⁵ sind gleich oder verschieden und besonders bevorzugt ein Wasserstoffatom, Methyl, Ethyl, Propyl, Phenyl, Benzyl, Tolyl, Vinyl oder Trimethylsilyl, insbesondere ein Wasserstoffatom.
R⁴ ist besonders bevorzugt ein Wasserstoffatom oder eine C₁-C₁₀-Alkylgruppe, wie Methyl, Ethyl, Propyl, Butyl oder Heptyl.
R⁶, R⁷, R⁸ und R¹¹ sind gleich oder verschieden und besonders bevorzugt ein Wasserstoffatom, eine C₁-C₁₀-Alkylgruppe wie Methyl, Ethyl, Propyl, Butyl oder Heptyl oder eine C₆-C₁₀-Arylgruppe wie Phenyl oder Naphtyl.
R⁹ und R¹⁰ sind gleich oder verschieden und besonders bevorzugt ein Wasserstoffatom oder eine C₁-C₁₀-Alkylgruppe wie Methyl, Ethyl, Propyl, Butyl oder Heptyl, oder R⁹ und R¹⁰ zusammen mit den sie verbindenden Atomen bilden ein Ringsystem.
R^{3'} und R^{5'} sind gleich oder verschieden und besonders bevorzugt ein Wasserstoffatom, Methyl, Ethyl, Propyl, Phenyl, Benzyl, Tolyl, Vinyl oder Trimethylsilyl, insbesondere ein Wasserstoffatom.
R^{4'} ist besonders bevorzugt ein Wasserstoffatom oder eine C₁-C₁₀-Alkylgruppe, wie Methyl, Ethyl, Propyl, Butyl oder Heptyl.
R^{6'}, R^{7'}, R^{8'} und R^{11'} sind gleich oder verschieden und besonders bevorzugt ein Wasserstoffatom, eine C₁-C₁₀-Alkylgruppe wie Methyl, Ethyl, Propyl, Butyl oder Heptyl oder eine C₆-C₁₀-Arylgruppe wie Phenyl oder Naphtyl.
R^{9'} und R^{10'} sind gleich oder verschieden und besonders bevorzugt ein Wasserstoffatom oder eine C₁-C₁₀-Alkylgruppe wie Methyl, Ethyl, Propyl, Butyl oder Heptyl, oder R^{9'} und R^{10'} zusammen mit den sie verbindenden Atomen bilden ein Ringsystem.

Als Beispiele für erfindungsgemäße Metallocenverbindungen der Formel I seien genannt:
Bis-(2-methyl-4,5-benzoindenyl)zirkoniumdichlorid,
Bis-(4,5-benzoindenyl)zirkoniumdichlorid,
Bis-(2-methyl-*α*-acenaphtylindenyl)zirkoniumdichlorid,
Bis-(2-ethyl-α-acenaphtylindenyl)zirkoniumdichlorid,
Bis(-α-acenaphtylindenyl)zirkoniumdichlorid,
(2-methyl-4,5-benzoindenyl)(4,5-benzoindenyl)zirkoniumdichlorid,
(2-methyl-4,5-benzoindenyl)(2-methyl-α-acenaphtylindenyl)zirkoniumdichlorid,
Bis-(2-methyl-4,5-benzoindenyl)titandichlorid,
Bis-(2-methyl-4,5-benzoindenyl)hafniumdichlorid,
Bis-(2-methyl-4,5-benzoindenyl)zirkoniumdimethyl,
Bis-(4,5-benzoindenyl)zirkoniumdimethyl.

Die erfindungsgemäßen Metallocenverbindungen können hergestellt werden, indem ein Inden z.B. der Formel la 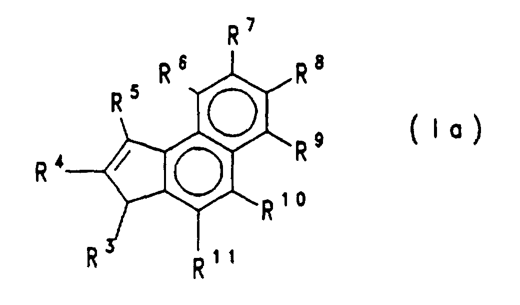 deprotoniert wird (z.B. mit Butyllithium) und mit einem Metallhalogenid wie ZrCl₄ umgesetzt wird.

Die vorliegende Erfindung betrifft auch ein Verfahren zur Herstellung eines Olefinpolymers durch Polymerisation mindestens eines Olefins in Gegenwart eines Katalysators, welcher mindestens eine Metallocenverbindung und einen Cokatalysator enthält, dadurch gekennzeichnet, daß das Metallocen eine Verbindung der Formel I ist. Unter dem Begriff Polymerisation wird eine Homopolymerisation wie auch eine Copolymerisation verstanden.

Bevorzugt werden Olefine der Formel R^{a}-CH=CH-R^{b} homo- oder copolymerisiert, worin R^{a} und R^{b} gleich oder verschieden sind und ein Wasserstoffatom oder einen Kohlenwasserstoffrest mit 1 bis 20 C-Atomen, insbesondere 1 bis 10 C-Atome, bedeuten, oder R^{a} und R^{b} zusammen mit den sie verbindenen Atomen einen oder mehrere Ringe bilden. Beispiele für solche Olefine sind 1-Olefine wie Ethylen, Propylen, 1-Buten, 1-Penten, 1-Hexen, 4-Methyl-1-penten oder 1-Octen, Styrol, Diene wie 1,3-Butadien oder 1,4-Hexadien und cyclische Olefine wie Norbornen, Tetracyclododecen, Norbornadien oder Vinylnorbornen. Bevorzugt wird in dem erfindungsgemäßen \ Verfahren Ethylen homopolymerisiert, oder Ethylen mit einem oder mehreren 1-Olefinen mit 3 bis 20 C-Atomen, wie Propylen, und/oder einem oder mehreren Dienen mit 4 bis 20 C-Atomen, wie 1,4-Butadien, copolymerisiert. Beispiele solcher Copolymere sind Ethylen/Propylen-Copolymere und Ethylen/Propylen/1,4-Hexadien-Copolymere.

Die Polymerisation wird bevorzugt bei einer Temperatur von -60 bis 250°C, besonders bevorzugt 50 bis 200°C, durchgeführt. Der Druck beträgt bevorzugt 0,5 bis 2000 bar, besonders bevorzugt 5 bis 64 bar.

Die Polymerisation kann in Lösung, in Masse, in Suspension oder in der Gasphase, kontinuierlich oder diskontinuierlich, ein- oder mehrstufig durchgeführt werden. Eine bevorzugte Ausführungsform ist die Gasphasenpolymerisation.

Bevorzugt enthält der in dem erfindungsgemäßen Verfahren eingesetzte Katalysator eine Metallocenverbindung der Formel I. Es können auch Mischungen zweier oder mehrerer Metallocenverbindungen der Formel I, oder Mischungen von Metallocenverbindungen der Formel I mit verbrückten Metallocenen eingesetzt werden, z.B. zur Herstellung von Polyolefinen mit breiter oder multimodaler Molmassenverteilung.

Prinzipiell ist als Cokatalysator in dem erfindungsgemäßen Verfahren jede Verbindung geeignet, die aufgrund ihrer Lewis-Acidität das neutrale Metallocen in ein Kation überführen und dieses stabilisieren kann ("labile Koordination"). Darüber hinaus soll der Cokatalysator oder das aus ihm gebildete Anion keine weiteren Reaktionen mit dem gebildeten Metallocenkation eingehen (EP 427 697). Als Cokatalysator wird bevorzugt eine Aluminiumverbindung und/oder eine Borverbindung verwendet.

Die Borverbindung hat bevorzugt die Formel R¹²ₓNH₄₋ₓBR¹³₄, R¹²ₓPH₄₋ₓBR¹³₄, R¹²₃CBR¹³₄ oder BR¹³₃, worin x eine Zahl von 1 bis 4, bevorzugt 3, bedeutet, die Reste R¹² gleich oder verschieden, bevorzugt gleich sind, und C₁-C₁₀-Alkyl oder C₆-C₁₈-Aryl sind, oder zwei Reste R¹² zusammen mit dem sie verbindenden Atomen einen Ring bilden, und die Reste R¹³ gleich oder verschieden, bevorzugt gleich sind, und C₆-C₁₈-Aryl sind, das durch Alkyl, Haloalkyl oder Fluor substituiert sein kann. Insbesondere steht R¹² für Ethyl, Propyl, Butyl oder Phenyl und R¹³ für Phenyl, Pentafluorophenyl, 3,5-Bistrifluoromethylphenyl, Mesityl, Xylyl oder Tolyl (EP 277 003, EP 277 004 und EP 426 638).

Bevorzugt wird als Cokatalysator eine Aluminiumverbindung wie Aluminoxan und/oder ein Aluminiumalkyl eingesetzt.

Besonders bevorzugt wird als Cokatalysator ein Aluminoxan, insbesondere der Formel IIa für den linearen Typ und/oder der Formel IIb für den cyclischen Typ verwendet, 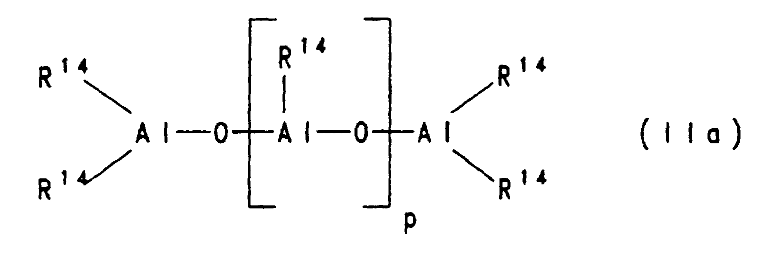 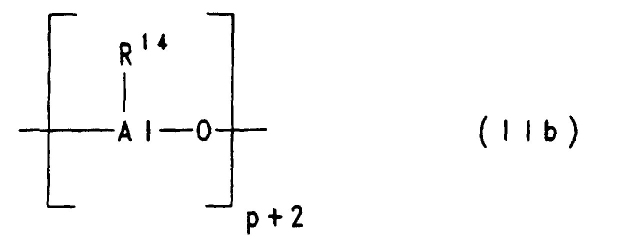 , wobei in den Formeln IIa und IIb die Reste R¹⁴ gleich oder verschieden sind und Wasserstoff oder eine C₁-C₂₀-Kohlenwasserstoffgruppe wie eine C₁-C₁₈-Alkylgrupppe, eine C₆-C₁₈-Arylgruppe oder Benzyl bedeuten, und p eine ganze Zahl von 2 bis 50, bevorzugt 10 bis 35 bedeutet.

Bevorzugt sind die Reste R¹⁴ gleich und bedeuten Wasserstoff, Methyl, Isobutyl, Phenyl oder Benzyl, besonders bevorzugt Methyl.

Sind die Reste R¹⁴ verschieden, so sind sie bevorzugt Methyl und Wasserstoff oder alternativ Methyl und Isobutyl, wobei Wasserstoff oder Isobutyl bevorzugt in einem zahlenmäßigen Anteil von 0,01 bis 40 % (der Reste R¹⁴) enthalten sind.

Die Verfahren zur Herstellung der Aluminoxane sind bekannt (DE 4 004 477).

Die genaue räumliche Stuktur der Aluminoxane ist nicht bekannt (J. Am. Chem. Soc. (1993) 115, 4971). Beispielsweise ist denkbar, daß sich Ketten und Ringe zu größeren zweidimensionalen oder dreidimensionalen Strukturen verbinden.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist möglich, die erfindungsgemäße Metallocenverbindung vor dem Einsatz in der Polymerisationsreaktion mit einem Cokatalysator, insbesondere einem Aluminoxan vorzuaktivieren. Dadurch wird die Polymerisationsaktivität deutlich erhöht. Die Voraktivierung der Metallocenverbindung wird vorzugsweise in Lösung vorgenommen. Bevorzugt wird dabei die Metallocenverbindung in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.%, jeweils bezogen auf die Gesamtlösungsmenge. Das Metallocen kann in der gleichen Konzentration eingesetzt werden, vorzugsweise wird es jedoch in einer Menge von 10⁻⁴ - 1 mol pro mol Aluminoxan eingesetzt. Die Voraktivierungsdauer beträgt 5 Minuten bis 60 Stunden, vorzugsweise 5 bis 60 Minuten. Man arbeitet bei einer Temperatur von -78 bis 100°C, vorzugsweise 0 bis 70°C.

Dabei wird die Metallocenverbindung bevorzugt in einer Konzentration, bezogen auf das Übergangsmetall, von 10⁻³ bis 10⁻⁸, vorzugsweise 10⁻⁴ bis 10⁻⁷ mol Übergangsmetall pro dm³ Lösemittel bzw. pro dm³ Reaktorvolumen angewendet. Das Aluminoxan, wird bevorzugt in einer Konzentration von 10⁻⁶ bis 10⁻¹ mol, vorzugsweise 10⁻⁵ bis 10⁻² mol pro dm³ Lösemittel bzw. pro dm³ Reaktorvolumen verwendet. Die anderen genannten Cokatalysatoren werden in etwa äquimolaren Mengen zu der Metallocenverbindung verwendet. Prinzipiell sind aber auch höhere Konzentrationen möglich.

Zur Entfernung von im Olefin vorhandenen Katalysatorgiften ist eine Reinigung mit einer Aluminiumverbindung, bevorzugt einem Aluminiumalkyl, wie Trimethylaluminium oder Triethylaluminium, vorteilhaft. Diese Reinigung kann sowohl im Polymerisationssystem selbst erfolgen, oder das Olefin wird vor der Zugabe in das Polymerisationssystem mit der Aluminiumverbindung in Kontakt gebracht und anschließend wieder abgetrennt.

Als Molmassenregler und/oder zur Steigerung der Aktivität kann in dem erfindungsgemäßen Verfahren Wasserstoff zugegeben werden. Hierdurch können niedermolekulare Polyolefine wie Wachse erhalten werden.

Bevorzugt wird in dem erfindungsgemäßen Verfahren die Metallocenverbindung mit dem Cokatalysator außerhalb des Polymerisationsreaktors in einem separaten Schritt unter Verwendung eines geeigneten Lösemittels umgesetzt. Dabei kann eine Trägerung vorgenommen werden.

Im dem erfindungsgemäßen Verfahren kann mit Hilfe der Metallocenverbindung eine Vorpolymerisation erfolgen. Zur Vorpolymerisation wird bevorzugt das (oder eines der) in der Polymerisation eingesetzte(n) Olefin(e) verwendet.

Der in dem erfindungsgemäßen Verfahren eingesetzte Katalysator kann geträgert sein. Durch die Trägerung läßt sich beispielsweise die Kornmorphologie des hergestellten Polyolefins steuern. Dabei kann die Metallocenverbindung zunächst mit dem Träger und anschließend mit dem Cokatalysator umgesetzt werden. Es kann auch zunächst der Cokatalysator geträgert werden und anschließend mit der Metallocenverbindung umgesetzt werden. Auch ist es möglich das Reaktionsprodukt von Metallocenverbindung und Cokatalysator zu trägern. Geeignete Trägermaterialien sind beispielsweise Silikagele, Aluminiumoxide, festes Aluminoxan oder andere anorganische Trägermaterialien wie beispielsweise Magnesiumchlorid. Ein geeignetes Trägermaterial ist auch ein Polyolefinpulver in feinverteilter Form. Die Herstellung des geträgerten Cokatalysators kann beispielsweise wie in EP 567 952 beschrieben durchgeführt werden.

Wenn die Polymerisation als Suspensions- oder Lösungspolymerisation durchgeführt wird, wird ein für das Ziegler-Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Hexan, Heptan, Isooctan, Cyclohexan, Methylcyclohexan, genannt. Weiterhin kann eine Benzin- bzw. hydrierte Dieselölfraktion benutzt werden. Brauchbar ist auch Toluol.

Werden inerte Lösemittel verwendet, werden die Monomeren gasförmig oder flüssig zudosiert.

Die Dauer der Polymerisation ist beliebig, da das in dem erfindungsgemäßen Verfahren zu verwendende Katalysatorsystem einen nur geringen zeitabhängigen Abfall der Polymerisationsaktivität zeigt.

Die nach dem erfindungsgemäßen Verfahren hergestellten Polymere sind insbesondere zur Herstellung von Formkörpern wie Folien, Platten oder Großhohlkörpern (z.B. Rohre) geeignet.

Die erfindungsgemäße Metallocenverbindung zeichnet sich dadurch aus, daß der synthetische Aufwand der Einführung einer Verbrückung zwischen den Liganden entfällt. Hierdurch wird die Herstellung der Metallocene verbilligt und Ausbeuteverluste vermieden.

Außerdem weisen sowohl die rac- wie auch die meso-Form der erfindungsgemäßen Metallocenverbindung eine vergleichbare Polymerisationsaktivität und auch Comonomer-Einbaurate auf, so daß auf die aufwendige Trennung von rac- und meso-Form verzichtet werden kann, insbesondere wenn die Metallocenverbindung zur Copolymerisation eingesetzt wird.

Die erfindungsgemäße Metallocenverbindung kann vorteilhaft zur Herstellung von Copolymeren, insbesondere ethylenhaltigen Copolymeren, mit niedriger Dichte wie LLDPE eingesetzt werden. Insbesondere eignet sich die erfindungsgemäße Metallocenverbindung zur Herstellung von Copolymeren, insbesondere ethylenhaltigen Copolymeren, mit niedriger Dichte unter Verwendung niedriger Comonomer-Konzentrationen. Dies ist insbesondere dann von Vorteil, wenn aus technischen oder ökonomischen Erwägungen eine niedrige Comonomerkonzentration einzuhalten ist, z.B. wenn in der Gasphasenpolymerisation Comonomere bei Überschreiten der Sättigungskonzentration kondensieren und technische Schwierigkeiten verursachen. Insbesondere vorteilhaft ist die Verwendung der erfindungsgemäßen Metallocenverbindung bei der Copolymerisation mit höhersiedenden Comonomeren in der Gasphasenpolymerisation.

Die erfindungsgemäße Metallocenverbindung weist eine hohe Comonomer-Einbaurate auf und eignet sich zur Herstellung comonomerreicher Copolymere wie Elastomere.

### Beispiele:

### Folgende Beispiele sollen die Erfindung näher erläutern:

Herstellung und Handhabung organometallischer Verbindungen erfolgten unter Ausschluß von Luft und Feuchtigkeit unter Argon-Schutzgas (Schlenk-Technik). Alle benötigten Lösungsmittel wurden vor Gebrauch durch mehrstündiges Sieden über einem geeigneten Trockenmittel und anschließende Destillation unter Argon absolutiert.

Die Verbindungen wurden mit ¹H-NMR, ¹³C-NMR- und IR-Spektroskopie charakterisiert.

Die angegebenen Polymerschmelzpunkte Tₘ sind einer DSC-Messung für das zweite Aufschmelzen bei einer Aufheizrate von 10°C/min entnommen.

Toluol lösliches Methylaluminoxan wird als 10 %ige Toluol Lösung von der Fa. WITCO bezogen und enthält gemäß Aluminium-Bestimmung 36 mg Al/ml.

Die Bestimmung des Comonomer-Einbaus erfolgt mit ¹³C-NMR gemäß der Methode von Randall (Macromolecules 1994, 27, 2120).

Die Bestimmung der Dichte erfolgt nach Gradientenmethode gemäß ISO 1183-1987.

### Beispiel 1

### Bis-(2-methyl-4,5-benzoindenyl)zirkoniumdichlorid:

In 500 ml Toluol werden 30 g (166 mmol) 2-Methyl-4,5-benzoinden gelöst und mit 62 ml (166 mmol) einer 2,68 molaren Butyllithium-Lösung in Toluol versetzt. Die Suspension wird noch 1 h bei 60°C nachgerührt und anschließend auf -30°C abgekühlt. 29,7 g (78,9 mmol) ZrCl₄*2THF werden zugegeben und man läßt die Reaktionsmischung in 12 h auf Raumtemperatur erwärmen. Die gelbfarbene Suspension wird über eine Glasfritte filtriert und mit 200 ml THF gewaschen.
Vom Filtrat wird im Vakuum das Lösungsmittel auf die Hälfte seines Volumens reduziert und die Lösung zur Kristallisation bei -30°C gelagert. Der ausgefallene Niederschlag wird abfiltriert und das Filtrat nochmals auf die Hälfte seines Volumens reduziert. Nach erneuter Kristallisation erhält man 9,1 g (22 %) meso-Bis-(2-methyl-4,5-benzoindenyl)zirkoniumdichlorid mit weniger als 10 % der racemischen Verbindung.

Der Rückstand auf der Glasfritte wird mit 500 ml Methylenchlorid gewaschen und der verbleibende Rückstand im Vakuum getrocknet. Man erhält 7,3 g (18 %) rac-Bis-(2-methyl-4,5-benzoindenyl)zirkoniumdichlorid als zitronengelber Feststoff.

### Beispiel 2:

500 ml Dieselöl (Sdp. 100-120°C), 20 ml Hexen und 10 ml 10 Gew.-% Lösung von Methylaluminoxan in Toluol wurden in einem Laborautoklaven unter Stickstoff vorgelegt und auf 70°C unter 700 Upm Rühren temperiert. Parallel hierzu wurden 0,1 mg rac-Bis-(2-methyl-4,5-benzoindenyl)zirkoniumdichlorid in 1 ml 10 Gew.-% MAO-Lösung in Toluol gelöst.
Die Polymerisation wird durch Zugabe der Metallocen/MAO-Lösung und durch Aufpressen von 4 bar Ethylen gestartet. Nach 15 min wird die Polymerisation mit CO₂ gestoppt und der Reaktor-Inhalt in 200 ml methanolische HCI abgelassen. Das Gemisch wird 5 h zur Entfernung von Aluminium gerührt, das Polymer anschließend abfiltriert und mit Wasser sowie Aceton gewaschen und zur Bestimmung der Ausbeute 12 h im Vakuum bei 120°C getrocknet. Die Menge Metallocen wurde so gewählt, daß nicht mehr als 5 g Polymer resultierten. Eine 1 g Probe wird zur Entfernung von Rest-Comonomer in Dieselöl (Sdp. 100-120°C) in der Hitze gelöst, anschließend ausgefällt, abfiltiert, mit Aceton gewaschen und nochmals im Vakuum bei 120°C getrocknet. Die Polymerisationsergebnisse sind in Tabelle 1 angegeben.

### Beispiel 3

Beispiel 2 wurde wiederholt, wobei 0,1 mg meso-Bis-(2-methyl-4,5-benzoindenyl)zirkoniumdichlorid als Metallocen verwendet wurde. Die Polymerisationsergebnisse sind in Tabelle 1 angegeben.

### Beispiele 4 und 5 (Vergleichsbeispiele)

Beispiel 2 wurde mit 20 ml (Bsp. 4) bzw. 50 ml (Bsp. 5) Hexen unter Verwendung des Metallocens Bis(methylcyclopentadienyl)zirkoniumdichlorid wiederholt. Die Polymerisationsergebnisse sind in Tabelle 1 angegeben.

### Beispiel 6 (Vergleichsbeispiel)

Beispiel 2 wurde mit 20 ml Hexen unter Verwendung des Metallocens Ethylenbis(indenyl)zirkoniumdichlorid wiederholt. Polymerisationsergebnisse sind in Tabelle 1 angegeben.

**Tabelle 1**

| Bsp. | ml Hexen | mg Kat. | Ausbeute [g Polymer] | Tₘ[°C] | Dichte [g/dm³] | mol % Hexen |
|---|---|---|---|---|---|---|
| 2 | 20 | 0,1 | 5,6 | 102,5 | 0,905 | 6,1 |
| 3 | 20 | 0,1 | 5,5 | 102,6 | 0,905 | 6 |
| V4 | 20 | 0,1 | 5,2 | 123,8 | 0,927 | 1,6 |
| V5 | 50 | 0,1 | 2,1 | 122,9 | 0,911 | 1,6 |
| V6 | 20 | 0,1 | 3,8 | 106,6 | 0,909 | 5,5 |

### Beispiel 7:

Geträgertes Methylaluminoxan wird gemäß Beispiel 13 der EP-A-567 952 (auf die hiermit ausdrücklich Bezug genommen wird) als 13,2 Gew.-% Suspension in Decan hergestellt. Der Feststoff aus 39 ml der Suspension wird abfiltriert und mit 40 ml Dieselöl sowie 10 mg rac-Bis(2-methyl-4,5-benzoindenyl)zirkoniumdichlorid-Pulver versetzt, 2 h bei Normaltemperatur gerührt und anschließend filtriert.

Parallel hierzu wird ein 16 dm³ Reaktor mit 8 dm³ iso-Butan, 10 mmol Triisobutylaluminium in Dieselöl und 100 ml 1-Hexen gefüllt und bei 70°C Ethylen bis zu einem Druck von 30 bar dosiert.

Der Katalysator wird in Butan suspendiert eindosiert, die Temperatur auf 70°C geregelt und der Gesamtdruck durch Ethylen-Dosierung konstant gehalten. In Intervallen von 15 min werden jeweils 100 ml Hexen nachdosiert. Nach 1 h wird die Polymerisation mit CO₂-Gas gestoppt. Nach Entspannen und Öffnen des Reaktors werden 1,35 kg LLDPE vom Schüttgewicht 321 g/dm³ und einer Dichte von 0,9105 gefunden. Der Reaktor ist frei von Belägen.

### Beispiel 8:

5 mg rac-Bis(2-methyl-4,5-benzoindenyl)zirkoniumdichlorid werden in 20 ml 10 % Methylaluminoxan-Lösung in Toluol gelöst und 0,5 h gerührt.

Parallel hierzu wird ein 16 dm³ Reaktor mit 8 dm³ Propylen und mit 10 ml 10 Gew-% Triisobutylaluminium in Dieselöl gefüllt. Nach zudosieren der Katalysator-Lösung wird 1 h bei 70°C polymerisiert, mit C02 gestoppt, der Reaktor entspannt und geöffnet. Es resultieren 822 g PP mit Mₙ = 25100 g/mol und M_{w}/Mₙ = 2,2.

## Patentansprüche

1. Metallocenverbindung der Formel (I) 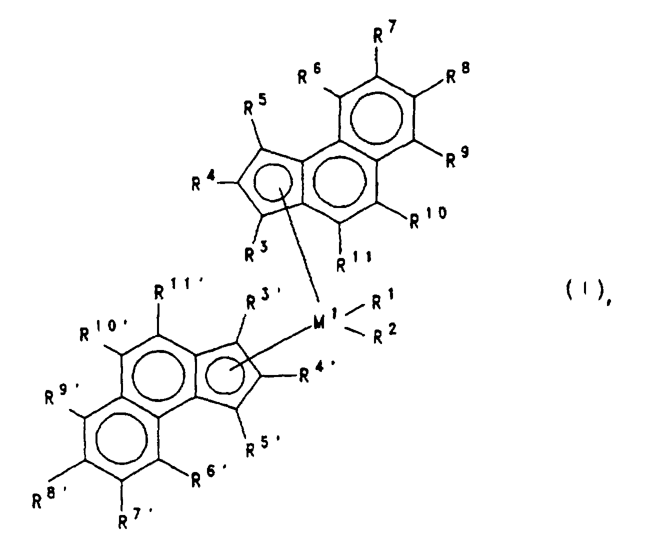 worin
M¹ ein Metall der Gruppe IVb, Vb oder Vlb des Periodensystems ist,
R¹ und R² gleich oder verschieden sind und ein Wasserstoffatom, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₈-C₄₀-Arylalkenylgruppe, eine OH-Gruppe oder ein Halogenatom bedeuten,
die Reste R³, R⁴, R⁵, R⁶, R⁷, R⁸, R⁹, R¹⁰ und R¹¹ gleich oder verschieden sind und ein Wasserstoffatom, ein Halogenatom, einen C₁-C₂₀-Kohlenwasserstoffrest, der halogeniert sein kann, einen -NR₂, -SR, -OR, -OSiR₃, -SiR₃ oder PR₂-Rest bedeuten, worin R ein Halogenatom, eine C₁-C₁₀-Alkylgruppe oder eine C₆-C₁₀-Arylgruppe ist, oder zwei oder mehr der Reste R³, R⁴, R⁵, R⁶, R⁷, R⁸, R⁹, R¹⁰ und R¹¹ zusammen mit den sie verbindenden Atomen ein Ringsystem bilden, und
die Reste R^{3'}, R^{4'}, R^{5'}, R^{6'}, R^{7'}, R^{8'}, R^{9'}, R^{10'} und R^{11'} gleich oder verschieden sind und ein Wasserstoffatom, ein Halogenatom, einen C₁-C₂₀-Kohlenwasserstoffrest, der halogeniert sein kann, einen -NR₂, -SR, -OR, -OSiR₃, -SiR₃ oder PR₂-Rest bedeuten, worin R ein Halogenatom, eine C₁-C₁₀-Alkylgruppe oder eine C₆-C₁₀-Arylgruppe ist, oder zwei oder mehr der Reste R^{3'}, R^{4'}, R^{5'}, R^{6'}, R^{7'}, R^{8'}, R^{9'}, R^{10'} und R^{11'} zusammen mit den sie verbindenden Atomen ein Ringsystem bilden.

2. Katalysator, enthaltend mindestens eine Metallocenverbindung gemäß Anspruch 1 und einen Cokatalysator.

3. Katalysator gemäß Anspruch 2, worin der Cokatalysator ein Aluminoxan ist.

4. Katalysator gemäß Anspruch 2 oder 3, **dadurch gekennzeichnet, daß** der Katalysator geträgert und/oder vorpolymerisiert ist.

5. Verfahren zur Herstellung eines Olefinpolymers durch Polymerisation mindestens eines Olefins in Gegenwart eines Katalysators gemäß einem oder mehreren der Ansprüche 2 bis 4.

6. Verwendung einer Metallocenverbindung der Formel I gemäß Anspruch 1 zur Olefin-polymerisation.

## Claims

1. A metallocene compound of the formula (I) 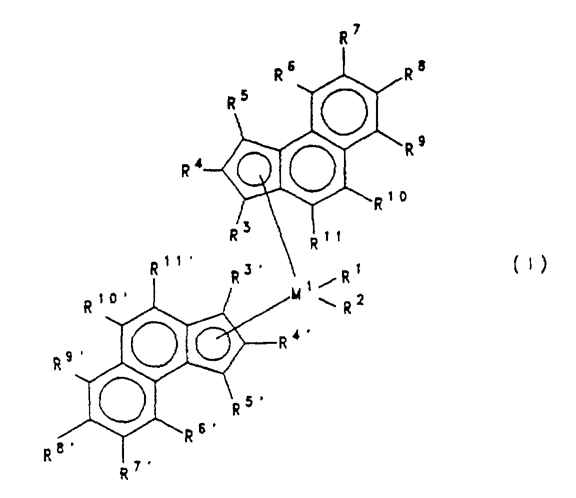 where
M¹ is a metal of group IVb, Vb or VIb of the Periodic Table,
R¹ and R² are identical or different and are each a hydrogen atom, a C₁-C₁₀-alkyl group, a C₁-C₁₀-alkoxy group, a C₆-C₁₀-aryl group, a C₆-C₁₀-aryloxy group, a C₂-C₁₀-alkenyl group, a C₇-C₄₀-arylalkyl group, a C₈-C₄₀-arylalkenyl group, an OH group or a halogen atom,
the radicals R³, R⁴, R⁵, R⁶, R⁷, R⁸, R⁹, R¹⁰ and R¹¹ are identical or different and are each a hydrogen atom, a halogen atom, a C₁-C₂₀-hydrocarbon radical which may be halogenated, an -NR₂, -SR, -OR, -OSiR₃, -SiR₃ or PR₂ radical, where R is a halogen atom, a C₁-C₁₀-alkyl group or a C₆-C₁₀-aryl group, or two or more of the radicals R³, R⁴, R⁵, R⁶, R⁷, R⁸, R⁹, R¹⁰ and R¹¹ together with the atoms connecting them form a ring system, and
the radicals R^{3'}, R^{4'}, R^{5'}, R^{6'}, R^{7'}, R^{8'}, R^{9'}, R^{10'} and R^{11'} are identical or different and are each a hydrogen atom, a halogen atom, a C₁-C₂₀-hydrocarbon radical which may be halogenated, an -NR₂, -SR, -OR, -OSiR₃, -SiR₃ or PR₂ radical, where R is a halogen atom, a C₁-C₁₀-alkyl group or a C₆-C₁₀-aryl group, or two or more of the radicals R^{3'}, R^{4'}, R^{5'}, R^{6'}, R^{7'}, R^{8'}, R^{9'}, R^{10'} and R^{11'} together with the atoms connecting them form a ring system.

2. A catalyst comprising at least one metallocene compound as claimed in claim 1 and a cocatalyst.

3. A catalyst as claimed in claim 2, wherein the cocatalyst is an aluminoxane.

4. A catalyst as claimed in claim 2 or 3, wherein the catalyst is supported and/or prepolymerized.

5. A process for preparing an olefin polymer by polymerization of at least one olefin in the presence of a catalyst as claimed in one or more of claims 2 to 4.

6. Use of a metallocene compound of the formula I as claimed in claim 1 for olefin polymerization.

## Revendications

1. Composé métallocène de formule (I) 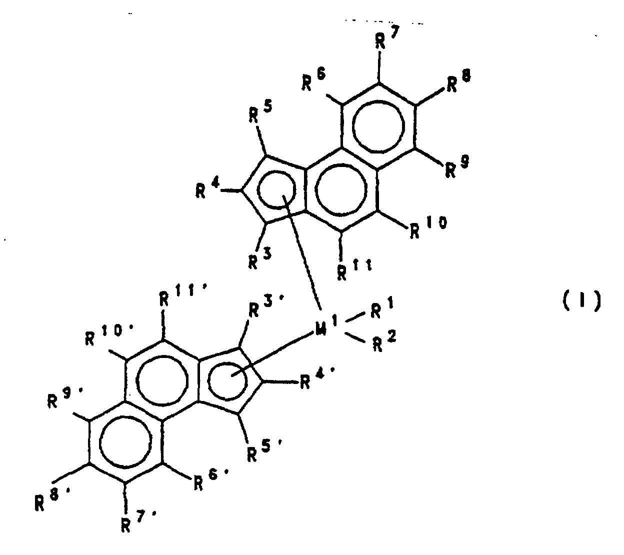 dans laquelle
M¹ est un métal du groupe IVb, Vb ou VIb du Tableau Périodique,
R¹ est R² sont identiques ou différents et représentent chacun un atome d'hydrogène, un groupe alkyle en C₁-C₁₀, un groupe alcoxy en C₁-C₁₀, un groupe aryle en C₆-C₁₀, un groupe aryloxy en C₆-C₁₀, un groupe alcényle en C₂-C₁₀, un groupe arylalkyle en C₇-C₄₀, un groupe arylalcényle en C₈-C₄₀, un groupe OH ou un atome d'halogène,
les radicaux R³, R⁴, R⁵, R⁶, R⁷, R⁸, R⁹, R¹⁰ et R¹¹ sont identiques ou différents et représentent chacun un atome d'hydrogène, un atome d'halogène, unradical hydrocarboné en C₁-C₂₀ pouvant être halogéné, un radical -NR₂, -SR, -OR, -OSiR₃, -SiR₃ ou PR₂ où R est un atome d'halogène, un groupe alkyle en C₁-C₁₀ ou un groupe aryle en C₆-C₁₀, ou encore au moins deux des radicaux R³, R⁴, R⁵, R⁶, R⁷, R⁸, R⁹, R¹⁰ et R¹¹, avec les atomes qui les relient, forment un système cyclique, et
les radicaux R^{3'}, R^{4'}, R^{5'}, R^{6'}, R^{7'}, R^{8'}, R^{9'}, R^{10'} et R^{11'} sont identiques ou différents et représentent chacun un atome d'hydrogène, un atome d'halogène, un radical hydrocarboné en C₁-C₂₀ pouvant être halogéné, un radical -NR₂, -SR, -OR, -OSiR₃, -SiR₃ ou PR₂ où R est un atome d'halogène, un groupe alkyle en C₁-C₁₀ ou un groupe aryle en C₆-C₁₀, ou encore au moins deux des radicaux R^{3'}, R^{4'}, R^{5'}, R^{6'}, R^{7'}, R^{8'}, R^{9'}, R^{10'} et R^{11'}, avec les atomes qui les relient, forment un système cyclique.

2. Catalyseur contenant au moins un composé métallocène selon la revendication 1 et un co-catalyseur.

3. Catalyseur selon la revendication 2, dans lequel le co-catalyseur est un aluminoxane.

4. Catalyseur selon la revendication 2 ou 3, **caractérisé en ce que** le catalyseur est supporté et/ou prépolymérisé.

5. Procédé de préparation d'un polymère d'oléfine par polymérisation d'au moins une oléfine en présence d'un catalyseur selon l'une ou plusieurs des revendications 2 à 4.

6. Utilisation d'un composé métallocène de formule I selon la revendication 1 pour la polymérisation d'oléfines.
